# EUROPEAN PATENT SPECIFICATION

(11) **EP 3 305 339 B1**
(45) Date of publication and mention of the grant of the patent: **05.05.2021**
(21) Application number: 15894325.8
(22) Date of filing: 23.06.2015
(51) Int. Cl.: A61L 27/24, C08J 5/18

(54) **METHOD FOR MANUFACTURING COLLAGEN FILM USING ULTRAVIOLET LIGHT, COLLAGEN FILM MANUFACTURED BY USING SAME, AND BIOMATERIAL PREPARED USING COLLAGEN FILM**
VERFAHREN ZUR HERSTELLUNG EINES KOLLAGENFILMS MITTELS ULTRAVIOLETTEM LICHT, DURCH VERWENDUNG DAVON HERGESTELLTER KOLLAGENFILM UND UNTER VERWENDUNG DES KOLLAGENFILMS HERGESTELLTES BIOMATERIAL
PROCÉDÉ DE FABRICATION DE FILM DE COLLAGÈNE À L'AIDE DE LUMIÈRE ULTRAVIOLETTE, FILM DE COLLAGÈNE FABRIQUÉ A L'AIDE DE CE DERNIER, ET MATÉRIAU BIOLOGIQUE PRÉPARÉ À L'AIDE DU FILM DE COLLAGÈNE

(30) Priority: 03.06.2015 KR 20150078507
(43) Date of publication of application: 11.04.2018
(73) Proprietor: Sewon Cellontech Co., Ltd, Seoul 150-724 (KR)
(72) Inventor: LEE, Joon Ho, Seoul 138-872 (KR); YOO, Ji Chul, Namyangju-si Gyeonggi-do 472-869 (KR); LEE, Myoung Sung, Bucheon-si Gyeonggi-do 420-850 (KR); KIM, Ki Soo, Seoul 136-075 (KR); LEE, Jun Keun, Seoul 131-816 (KR); SUH, Dong Sam, Seoul 139-924 (KR); CHANG, Cheong Ho, Seoul 135-841 (KR)
(74) Representative: dompatent von Kreisler Selting Werner - Partnerschaft von Patent- und Rechtsanwälten mbB
(86) International application number: PCT/KR2015/006356
(87) International publication number: WO 2016/195152

(56) References cited:
- EP-A1- 2 610 335
- EP-A2- 0 246 013
- WO-A1-2011/074208
- KR-A- 20050 014 390
- KR-A- 20130 108 269
- KR-A- 20150 053 606
- KR-B1- 100 791 512
- US-A1- 2006 177 492

## Description

### Technical Field

The present invention relates to a method of manufacturing a collagen film using ultraviolet (UV) light, a collagen film manufactured thereby, and a biomaterial produced using the collagen film, and more particularly to a method of manufacturing a collagen film using UV light, a collagen film manufactured thereby, and a biomaterial produced using the collagen film, in which collagen isolated from animal tissues is crosslinked using UV light to form a film.

### Background Art

Biomaterial may be defined as artificial material that is intermittently or constantly in contact with surrounding tissues *in vivo* and is exposed to body fluids in order to replace the functions of damaged or defective human tissues and organs.

Biomaterial for use in the human body has to be similar to portions of tissues to be restored from the viewpoint of size and shape, and the mechanical and physical properties thereof also have to be as close as possible to those of the original tissues in order to maintain sustained effects.

Thus, biomaterial is produced in various shapes so as to be adapted for the region of application and the purpose of use, and moreover, is manufactured in a dosage form of a solution, a film, a sponge, a fiber, or a hydrogel when using a biological polymer.

As products suitable for individual dosage forms, the solution formulation may include a skin graft material, a coating agent, a drug delivery vehicle, and a matrix for cell culture, and the film formulation may be provided in the form of a wound dressing, a drug delivery agent, an anti-adhesion membrane, an eardrum or a cornea.

Also, the sponge formulation may be applied to a wound dressing, a styptic agent, artificial skin and an artificial organ, and the fiber formulation may be applied to a surgical suture. The hydrogel formulation may be applied to a contact lens, a soft tissue implant, and a matrix for cell culture.

The biomaterial in a dosage form of a solution, sponge or hydrogel may be applied to and commercialized in a variety of fields. For a film-type biomaterial, however, there is an urgent need for research and development and commercialization because of the lack of appropriate dosage forms for products.

The biomaterial in a film dosage form may be used as follows.

In the case where the epidermis is damaged over a wide area due to a burn or severe abrasion, a therapeutic method using a wound dressing or a method of grafting a cell membrane in which epithelial cells have been cultured is being used.

The biomaterial in a film dosage form is suitable for use for the purpose of enhancing the engraftment rate to the affected region of tissue by strengthening the epithelial cell layer or performing the role of a wound dressing.

In otorhinolaryngology, such a biomaterial may be appropriately provided in the form of a patch used in an eardrum patch procedure (a procedure in which a patch is placed on a marginal perforation of the eardrum to induce the regeneration of the epithelial layer along the patch), which may be applied to injured eardrums.

In ophthalmology, such a biomaterial is available as a lens form useful after corneal surgery or when corneal injury occurs, and in dentistry, it is available as a barrier membrane (to prevent the exposure of defective sites, to prevent the inflow of bacteria, and to ensure space suitable for functional reconstruction for bone regeneration) such as a film for use in guided tissue regeneration.

Moreover, the biomaterial may be medically used for a drug delivery agent, an anti-adhesion membrane, an artificial organ, and the like.

Considering that it is best to regenerate the original tissue in the event of injury to the human body, it is very reasonable to apply collagen among various biomaterials because collagen is a protein that is distributed in almost all tissues in the human body and is essential for building up the human body.

Since collagen has excellent biocompatibility and tissue affinity, low antigenicity, and good cell attachability, differentiation- and proliferation-promoting action, hemostatic effects, and absorption after complete decomposition *in vivo,* collagen has superb properties and is suitable for use in a medical material.

When collagen is used as a medical biomaterial, a crosslinking process has to be performed in order to maintain proper shape and strength depending on the region of application and the purpose of use.

Collagen crosslinking is largely classified into chemical crosslinking and physical crosslinking. A chemical crosslinking process may include the use of a crosslinking agent such as formaldehyde, glutaraldehyde, carbodiimide (EDC), or a polyepoxy compound. A physical crosslinking process may include dry heat treatment, UV irradiation, and gamma irradiation.

Korean Patent No. 10-0536478 granted to Lifecord International Co. Ltd. discloses a biodegradable polymer substrate for artificial organ production, in which lyophilized collagen is re-dissolved into a collagen solution at a low concentration of less than 1 to 2% and glycosaminoglycan is crosslinked to form a biodegradable collagen substrate in the form of a film.

Korean Patent No. 10-1434041 granted to Genewel Co. Ltd. discloses a regenerative membrane made from a biotissue-derived material, in which the biotissue-derived material is swollen, pulverized, filtered, and then chemically crosslinked with hexamethylene diisocyanate, an epoxy group, carbodiimide or acyl azide or thermally crosslinked at a vacuum level of 50 ∼ 100 torr at 50 ∼ 200°C to form a membrane.

EP 0 246 013 discloses a method of producing a collagen film comprising: UV crosslinking a collagen film hydrated in hydrochloric and phosphoric acid solution and having a pH 5-9.

Most currently useful collagen crosslinking methods, including the aforementioned methods, make it difficult to secure the safety of biomaterials owing to the use of a crosslinking material that may be left behind in the living body to thus exhibit toxicity. Also, since the crosslinking process has to be performed through several steps, such as re-dissolution after lyophilization and then further physical crosslinking, the production process is complicated, a long time is required, and energy consumption is large.

Hence, thorough research has been carried out in the present invention to solve these problems, and thus a method and conditions for manufacturing a collagen film from a collagen solution using only UV crosslinking, without a chemical crosslinking agent or a lyophilization process, have been established.

### Disclosure

### Technical Problem

The present invention is intended to provide a method of manufacturing a collagen film using UV light, in which a collagen solution may be crosslinked using only UV light without a chemical crosslinking agent or lyophilization to form a collagen film in a dosage form having a smooth surface.

In addition, the present invention is intended to provide a collagen film, manufactured by the method of the invention described above.

In addition, the present invention is intended to provide a biomaterial for medical use, produced using the collagen film.

### Technical Solution

Therefore, the present invention provides a method of manufacturing a collagen film as defined in claim 1.

In addition, the present invention provides a collagen film manufactured by the above method.

In addition, the present invention provides a biomaterial produced using the collagen film, the biomaterial being any one selected from among a wound dressing, an artificial eardrum patch, a dental barrier membrane, an artificial cornea, an artificial lens, an anti-adhesion membrane, a drug delivery agent, and an artificial organ.

### Advantageous Effects

According to the present invention, a collagen film in a dosage form having a smooth surface can be manufactured from collagen through UV irradiation.

Also, the collagen film can be utilized to produce a wound dressing, an artificial eardrum patch, an artificial cornea and lens, a dental barrier membrane, a drug delivery agent, an anti-adhesion membrane, and an artificial organ.

Also, the method of the invention obviates the need for a biotoxic chemical crosslinking agent and for processes such as lyophilization and dry heat treatment, and is thus efficient and industrially applicable.

### Description of Drawings

FIG. 1 shows a process of manufacturing a collagen film using UV light according to an embodiment of the present invention;
FIG. 2 is a photograph showing a collagen film manufactured using pig skin tissue according to the present invention;
FIG. 3 is a photograph showing a collagen film manufactured using skin tissue of duck's feet according to the present invention;
FIG. 4 is a photograph showing wound dressings in various sizes, produced using the collagen film according to the present invention;
FIG. 5 is a photograph showing dental barrier membranes in various sizes, produced using the collagen film according to the present invention;
FIG. 6 is a photograph showing collagen films having various shapes in order to demonstrate the flexibility of the collagen film according to the present invention; and
FIG. 7 is of photographs showing artificial eardrum patches, produced using the collagen film according to the present invention.

### [Brief Description of the Reference Numerals]

100: collagen film
S100: preparation of collagen solution
S200: defoaming
S300: crosslinking
S400: drying

### Best Mode

The present invention has been made keeping in mind the problems encountered in the related art, and addresses a method of manufacturing a collagen film using UV light, which may be performed through a simple process without the need for a chemical material or lyophilization and is thus efficient and industrially applicable.

In addition, the present invention addresses a biomaterial for medical use, produced using a collagen film manufactured by the above method.

In the present invention, collagen is a protein extracted from various animal tissues through acid treatment or treatment with an enzyme such as pepsin.

Hereinafter, embodiments of the present invention are described with reference to the appended drawings.

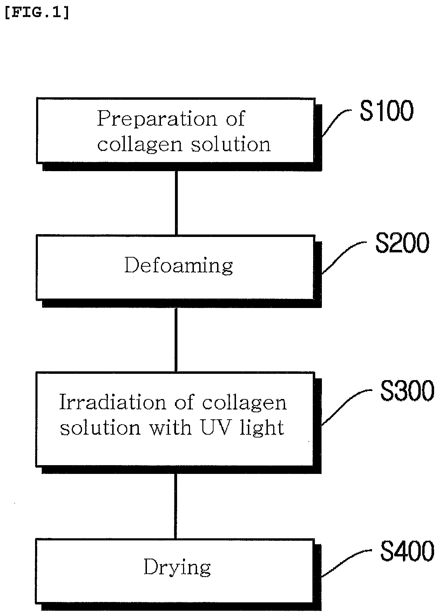

FIG. 1 shows a process of manufacturing a collagen film using UV light according to an embodiment of the present invention. With reference thereto, the method of manufacturing the collagen film using UV light is described below.

According to the present invention, a collagen film for medical use is manufactured by:
1) a collagen solution preparation step of preparing a collagen solution by dissolving collagen in an acidic solution (S100);
2) a crosslinking step of aliquoting the collagen solution into a mold and irradiating the aliquoted collagen solution with UV light so as to be physically crosslinked (S300); and
3) a drying step of naturally drying the crosslinked collagen (S400).

The steps of the method of the present invention are specified below.

First, a collagen solution is dissolved or diluted in the acidic solution in an amount of 0.2 ∼ 3% and preferably 0.6 ∼ 1%.

The acidic solution used to dissolve or dilute the collagen is an aqueous solution of phosphoric acid or hydrochloric acid at a pH of 2 ∼ 5 and preferably a pH of 2 - 3.

Collagen that is useful in the present invention includes various tissues of mammals, birds and fish.

The diluted collagen solution preferably contains no foam, but foaming may occur during the diluted collagen solution preparation step (S100).

Hence, defoaming the diluted collagen solution (S200) may be further performed.

Thereafter, the diluted collagen solution is aliquoted into a plastic mold.

In order to form a film having a thickness of 50 ∼ 100 µm using the diluted collagen solution, the collagen solution is aliquoted in an amount of 0.4 ∼ 1.8 mL/cm² and preferably 0.6 ∼ 1.2 mL/cm² into a mold.

If the thickness of the film is less than 50 µm, it is difficult to obtain a film from the mold and the resulting film may easily tear, and thus the control thereof becomes difficult.

On the other hand, if the thickness thereof exceeds 100 µm, the resulting film is relatively inflexible, thus limiting the range of application thereof, and may also easily break down, and thus the control thereof becomes difficult.

Next, the mold into which the diluted collagen solution is aliquoted is crosslinked with UV light at room temperature.

Here, the wavelength of UV light is 10 ∼ 380 nm and preferably 200 ∼ 280 nm.

The intensity of UV light that is applied is 0.10 ∼ 0.50 mW/cm², and preferably 0.35 ∼ 0.38 mW/cm².

The UV irradiation is performed for 12 ∼ 48 hr, and preferably 24 ∼ 26 hr.

The crosslinked collagen is naturally dried (S400), thus forming a collagen film.

The drying is performed without wind blowing and at 15 ∼ 24°C.

Also, the drying is performed for 72 ∼ 168 hr and preferably 96 ∼ 120 hr.

In the case where the drying is performed at a high temperature, denaturation of a collagen protein may occur, making it impossible to obtain desired effects and making it difficult to form a film having a smooth surface.

Also, if the drying is conducted using wind, the resulting film may be formed to a non-uniform thickness and may become opaque.

Also, a dewatering device for easily removing water may be additionally used.

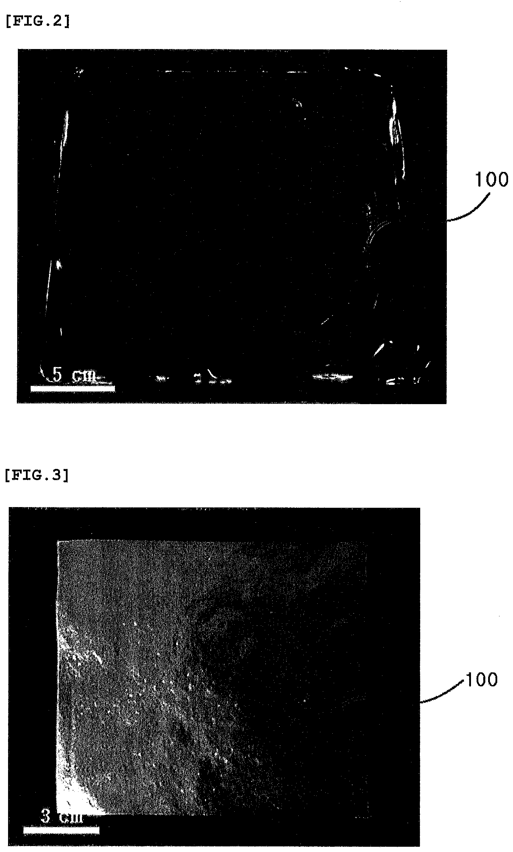

FIG. 2 is a photograph showing the collagen film manufactured using pig skin tissue according to the present invention, and FIG. 3 is a photograph showing the collagen film manufactured using skin tissue of duck's feet according to the present invention.

The collagen film 100 as shown in FIG. 2 or 3 may be obtained by the method of the invention, a better understanding of which will be given through the following [Example 1].

### [Example 1]

Collagen was isolated from pig skin tissue or skin tissue of duck's feet and was then dissolved and diluted to a collagen concentration of 0.6 ∼ 1% using an acidic solution at a pH of 2 ∼ 3, obtained by adding hydrochloric acid to distilled water.

Here, the concentration of collagen was measured using a hydroxyproline analysis method.

When foaming occurred upon the dissolution or dilution of collagen, the collagen solution was stored at 4°C for 24 hr and thus defoamed.

The defoamed collagen solution was aliquoted into a plastic mold.

In order to obtain a collagen film having a thickness of 50 ∼ 100 µm, the collagen solution was aliquoted in an amount of 0.6 ∼ 1.2 mL per unit area (cm²).

Next, the mold into which the collagen solution was aliquoted was irradiated with UV light at a wavelength of 253 nm at room temperature.

UV light was applied at an intensity of 0.35 ∼ 0.38 mW/cm² for 24 ∼ 26 hr to transfer energy of 30 ∼ 36 J/cm², thereby crosslinking collagen.

Next, the crosslinked collagen solution was dried in the mold.

The temperature of 15 ∼ 24°C was maintained for 96 ∼ 120 hr, and drying was performed without wind blowing in the absence of impurities.

After completion of the drying, as shown in FIGS. 2 and 3, the collagen film 100 in a dosage form having a smooth surface was manufactured.

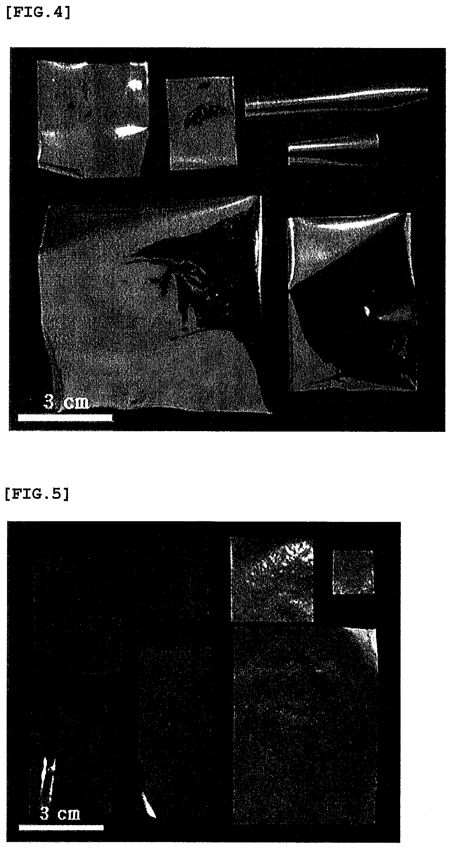

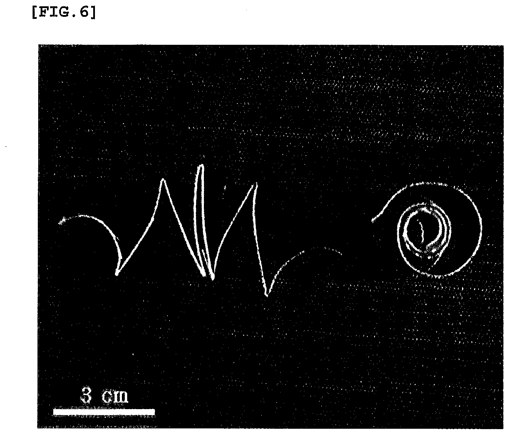

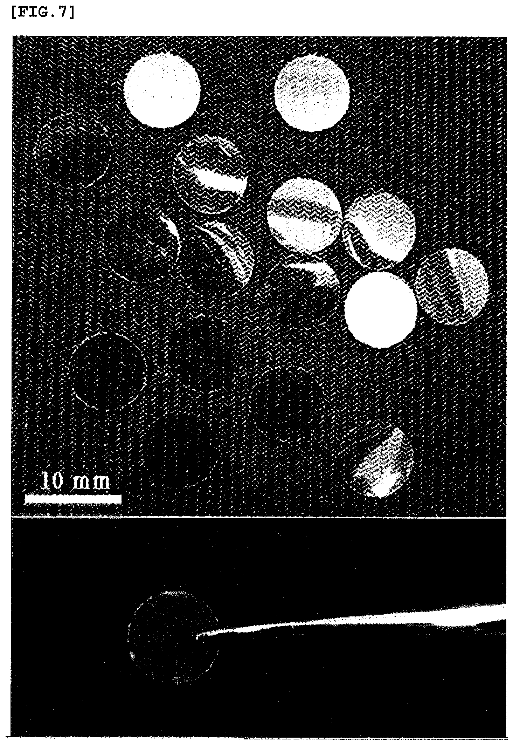

FIG. 4 is a photograph showing wound dressings in various sizes, produced using the collagen film according to the present invention, FIG. 5 is a photograph showing dental barrier membranes in various sizes, produced using the collagen film according to the present invention, FIG. 6 is a photograph showing collagen films having various shapes in order to demonstrate the flexibility of the collagen film according to the present invention, and FIG. 7 is of photographs showing artificial eardrum patches, produced using the collagen film according to the present invention.

The collagen film was manufactured through the above process, and the crosslinked and dried collagen film was separated from the mold in order to produce a variety of medical biomaterials.

The separated (stripped) collagen film 100 is cut to a predetermined size to thus produce a biomaterial of the present invention, such as a wound dressing, a dental barrier membrane, and an artificial eardrum patch, a better understanding of which will be given through the following [Example 2].

### [Example 2]

As shown in FIGS. 4 and 5, the collagen film was cut to various sizes in the range of 1 ∼ 20 cm using a surgical scalpel so as to have a size suitable for use as a wound dressing or a dental barrier membrane.

The collagen film 100 formed through UV irradiation may be cut into various shapes depending on the region of application or the purpose of use, and as shown in FIG. 6, the collagen film having high flexibility may even be used in the state of being bent or folded.

Also, as shown in FIG. 7, the collagen film was cut to a diameter of 8 mm and a thickness of 50 ∼ 100 µm using a biopsy punch so as to have a size suitable for use as an artificial eardrum patch.

The cut collagen film, having a smooth surface, was confirmed to be suitable for use in an eardrum patch procedure.

Moreover, the biomaterial produced using the collagen film of the present invention may be employed in an artificial cornea, an artificial lens, a drug delivery agent, an anti-adhesion membrane, an artificial organ, and the like, and may be used by being cut to various shapes and sizes depending on usage, and thus additional Examples therefor are omitted.

### Industrial Applicability

According to the present invention, a method of manufacturing a collagen film using UV light can be performed through a simple process without the additional use of a chemical material or without the need for processes such as lyophilization and dry heat treatment, and is thus efficient and industrially applicable.

Also, the collagen film of the present invention can be utilized in medical applications, such as a wound dressing, an artificial eardrum patch, an artificial cornea and lens, a dental barrier membrane, a drug delivery agent, an anti-adhesion membrane, an artificial organ, and the like.

## Claims

1. A method of manufacturing a collagen film using ultraviolet (UV) light, comprising:
a collagen solution preparation step of preparing a collagen solution by dissolving collagen in an acidic solution;
a crosslinking step of physically crosslinking the collagen solution through UV irradiation; and
a drying step of naturally drying the crosslinked collagen;
wherein the crosslinking step is performed using UV light at a wavelength of 10 ∼ 380 nm; and
wherein the UV light has an intensity of 0.10 ∼ 0.50 mW/cm²; and
wherein the UV light is applied for 12 ∼ 48 hr; and
wherein the step of naturally drying is performed at the temperature of 15 ∼ 24°C; and
wherein the step of naturally drying is performed without wind blowing.

2. The method of claim 1, further comprising defoaming the collagen solution dissolved in the acidic solution.

3. The method of claim 1, wherein the collagen is dissolved in an amount of 0.2 ∼ 3% in the acidic solution.

4. The method of claim 1, wherein the collagen is collagen isolated from any one selected from among a mammal, a bird and a fish.

5. The method of claim 1, wherein the acidic solution is an acidic solution at a pH of 2 ∼ 5.

6. The method of claim 5, wherein the acidic solution is a phosphoric acid or hydrochloric acid aqueous solution.

7. The method of claim 1, wherein the collagen solution obtained in the collagen solution preparation step is aliquoted in an amount of 0.4 ∼ 1.8 mL/cm² into a mold.

8. The method of claim 7, wherein the drying step is performed for 72 ∼ 168 hr.

9. A collagen film, manufactured by the method of any one of claims 1 to 8.

10. A biomaterial, produced using the collagen film of claim 9.

11. The biomaterial of claim 10, wherein the biomaterial is any one selected from among a wound dressing, an artificial eardrum patch, a dental barrier membrane, an artificial cornea, an artificial lens, an anti-adhesion membrane, a drug delivery agent, and an artificial organ.

## Patentansprüche

1. Verfahren zur Herstellung eines Collagenfilms unter Verwendung von ultraviolettem (UV) Licht, umfassend:
einen Collagenlösung-Herstellungsschritt, in dem eine Collagenlösung hergestellt wird, indem man Collagen in einer sauren Lösung auflöst;
einen Vernetzungsschritt, in dem die Collagenlösung durch UV-Bestrahlung physikalisch vernetzt wird; und
einen Trocknungsschritt, in dem das vernetzte Collagen in natürlicher Weise getrocknet wird;
wobei der Vernetzungsschritt durchgeführt wird, indem man UV-Licht mit einer Wellenlänge von 10 ∼ 380 nm verwendet; und
wobei das UV-Licht eine Intensität von 0,10 ∼ 0,50 mW/cm² aufweist; und
wobei das UV-Licht 12 ∼ 48 Stunden lang angewendet wird; und
wobei der Schritt des natürlichen Trocknens bei ein er Temperatur von 15 °C ∼ 24 °C durchgeführt wird; und
wobei der Schritt des natürlichen Trocknens ohne bewegte Luft durchgeführt wird.

2. Verfahren gemäß Anspruch 1, weiterhin umfassend das Entschäumen der Lösung des in der sauren Lösung gelösten Collagens.

3. Verfahren gemäß Anspruch 1, wobei das Collagen in einer Menge von 0,2% ∼ 3% in der sauren Lösung gelöst wird.

4. Verfahren gemäß Anspruch 1, wobei das Collagen ein Collagen ist, das aus einem Tier isoliert wurde, welches aus einem Säuger, einem Vogel und einem Fisch ausgewählt ist.

5. Verfahren gemäß Anspruch 1, wobei die saure Lösung eine saure Lösung mit einem pH-Wert von 2 ∼ 5 ist.

6. Verfahren gemäß Anspruch 5, wobei die saure Lösung eine wässrige Phosphorsäure- oder Chlorwasserstoffsäurelösung ist.

7. Verfahren gemäß Anspruch 1, wobei die in dem Collagenlösung-Herstellungsschritt erhaltene Collagenlösung in Aliquoten in einer Menge von 0,4 ∼ 1,8 ml/cm² in eine Form gegeben wird.

8. Verfahren gemäß Anspruch 7, wobei der Trocknungsschritt 72 ∼ 168 Stunden lang durchgeführt wird.

9. Collagenfilm, hergestellt nach dem Verfahren gemäß einem der Ansprüche 1 bis 8.

10. Biomaterial, hergestellt unter Verwendung des Collagenfilms gemäß Anspruch 9.

11. Biomaterial gemäß Anspruch 10, wobei das Biomaterial eines ist, das aus einem Wundverband, einem künstlichen Trommelfellpflaster, einer Zahnbarrieremembran, einer künstlichen Kornea, einer künstlichen Augenlinse, einer Antihaftmembran, einem Wirkstoffabgabemittel und einem künstlichen Organ ausgewählt ist.

## Revendications

1. Procédé pour fabriquer un film de collagène en utilisant de la lumière ultraviolette (UV), comprenant :
une étape de préparation d'une solution de collagène, dans laquelle une solution de collagène est préparée en dissolvant du collagène dans une solution acide,
une étape de réticulation, dans laquelle ladite solution de collagène est réticulée physiquement par rayonnement UV, et
une étape de séchage, dans laquelle ledit collagène réticulé est séché naturellement,
dans lequel l'étape de réticulation est effectuée en utilisant de la lumière UV d'une longueur d'onde de 10 ∼ 380 nm, et
dans lequel ladite lumière UV présente une intensité de 0,10 ∼ 0,50 mW/cm², et
dans lequel ladite lumière UV est appliquée pendant 12 ∼ 48 heures, et
dans lequel l'étape de séchage naturel est effectuée à une température de 15 ∼ 24 °C, et
dans lequel l'étape de séchage naturel est effectuée sans souffler de l'air.

2. Procédé selon la revendication 1, comprenant en outre le démoussage de la solution de collagène dissout dans la solution acide.

3. Procédé selon la revendication 1, dans lequel le collagène est dissout en une quantité de 0,2 ∼ 3 % dans la solution acide.

4. Procédé selon la revendication 1, dans lequel le collagène est du collagène isolé de l'un choisi parmi un mammifère, un oiseau et un poisson.

5. Procédé selon la revendication 1, dans lequel la solution acide est une la solution acide ayant un pH de 2 ∼ 5.

6. Procédé selon la revendication 5, dans lequel la solution acide est une solution aqueuse de l'acide phosphorique ou de l'acide chlorhydrique.

7. Procédé selon la revendication 1, dans lequel la solution de collagène obtenue dans l'étape de préparation d'une solution de collagène est aliquotée dans un moule en une quantité de 0,4 ∼ 1,8 ml/cm².

8. Procédé selon la revendication 7, dans lequel l'étape de séchage est effectuée pendant 72 ∼ 168 heures.

9. Film de collagène fabriqué par le procédé selon l'une quelconque des revendications 1 à 8.

10. Biomatériau produit en utilisant le film de collagène selon la revendication 9.

11. Biomatériau selon la revendication 10, dans lequel ledit biomatériau est l'un choisi parmi un pansement, une pièce de tympan artificiel, une membrane de barrière dentale, une cornée artificielle, un cristallin artificiel, une membrane anti-adhésion, un agent de délivrance de principe actif, et un organe artificiel.
